# EUROPEAN PATENT SPECIFICATION

(11) **EP 3 684 305 B1**
(45) Date of publication and mention of the grant of the patent: **10.09.2025**
(21) Application number: 18778993.8
(22) Date of filing: 21.09.2018
(51) Int. Cl.: A61F 5/445

(54) **A DRAINABLE OSTOMY APPLIANCE**
ENTLEERBARES OSTOMIEGERÄT
APPAREIL DE STOMIE DRAINABLE

(30) Priority: 22.09.2017 GB 201715401
(43) Date of publication of application: 29.07.2020
(73) Proprietor: Salts Healthcare Limited, West Midlands B7 4AA (GB)
(72) Inventor: TRETHEWAY, Lee, Birmingham West Midlands B7 4AA (GB); HOWARD, Lee, Birmingham West Midlands B7 4AA (GB); POWNER, Iain, Birmingham West Midlands B7 4AA (GB); ALFARO, Jesus, Birmingham West Midlands B7 4AA (GB)
(74) Representative: Forresters IP LLP
(86) International application number: PCT/GB2018/052693
(87) International publication number: WO 2019/058129

(56) References cited:
- WO-A1-03/086250
- WO-A1-2007/115574
- GB-A- 2 414 677
- US-A1- 2007 265 588

## Description

### Description of Invention

The invention relates to ostomy appliances. In particular, but not exclusively, the invention relates to drainable or ileostomy appliances.

It is known to provide drainable ostomy appliances with means to assist in opening and closing an opening thereof to permit the contents of the device to be emptied. For example, WO2003/086250 discloses an ostomy appliance including first and second walls and first and second stiffening strips. However, openings can become stuck in a closed condition, in use. This makes it difficult for a user to manipulate the opening. In particular, if stiffening members are used at or near the exit of the opening and the opening becomes stuck in a closed condition, users may find it difficult to open the opening by applying finger pressure alone to the ends of the stiffening members. This is because the stiffening members will all tend to bow in the same direction, resulting in the opening remaining closed. It can also become difficult to clean the opening of such an ostomy appliance effectively. Ineffective cleaning can result in the leakage of waste from the device, which is clearly undesirable.

The present invention seeks to address these problems.

The present invention relates to a drainable ostomy appliance as set out in the claims.

Further features of the various aspects of the invention are set out in the clauses which follow the description and the appended claims.

Embodiments of the invention will now be described by way of example only with reference to the accompanying drawings, of which:
Figure 1 is a front view of a first embodiment of an ostomy device in accordance with the invention with an outlet thereof shown in an unrolled condition;
Figure 2 is a front view of the device of figure 1 but with the outlet thereof shown in a rolled, i.e. closed, condition;
Figure 3 is a side cross-sectional view of the ostomy device corresponding to figure 1;
Figure 4 is a side cross-sectional view of the ostomy device corresponding to figure 2;
Figure 5 is an enlarged front view of a stiffening member and outlet of the device of figure 1;
Figure 6 is a perspective view showing end and side profiles of stiffening members of the ostomy device of figure 1;
Figure 7 is an end view showing the end profile of the stiffening members of the ostomy device of figure 1;
Figure 8 is a perspective view showing end profiles of the two stiffening members of the ostomy device of figure 1;
Figure 9 is a perspective view of the outlet of the ostomy device, showing the separation of the stiffening members from each other during emptying of the device;
Figure 10 is an end view (looking into an interior of the ostomy device) of the outlet of the ostomy device, showing the separation of the stiffening members from each other during emptying of the device;
Figure 11 is a further front view of the ostomy device of figure 1 with the outlet shown in an unrolled condition;
Figure 12 is a further enlarged front view of the stiffening member and outlet of figure 1;
Figure 13 is a side cross-sectional view of the stiffening members and outlet of a second embodiment of an ostomy device in accordance with the invention, with the outlet thereof shown in an unrolled condition;
Figure 14 is a side view of the device of figure 13 but with the outlet thereof shown in a rolled, i.e. closed, condition;
Figure 15 is a rear view of a third embodiment of an ostomy device in accordance with the invention with an outlet thereof shown in an unrolled condition; and
Figure 16 is a side cross-sectional view of the ostomy device of figure 15.

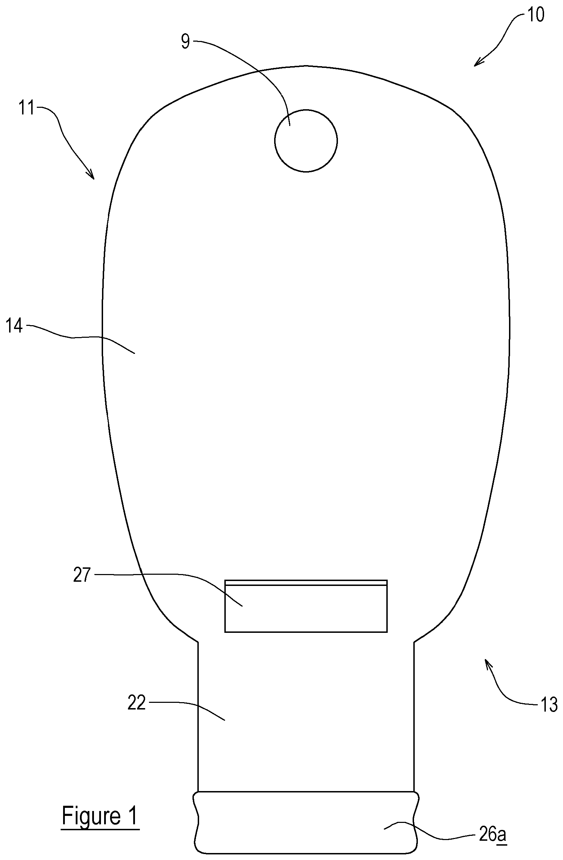

Referring to figures 1 to 12, these show a first embodiment of an ostomy appliance in accordance with the present invention, generally at 10. The ostomy appliance 10 has a top, or upper end, 11 and a bottom, or lower end, 13. The ostomy appliance 10 has first and second walls 12, 14 connected to each other at or near their peripheries, by any suitable means known in the art. For example, they could be adhered to each other or heat welded.

The first wall 12 includes a stoma-receiving opening 16 and supports a connection member or flange 20 for attaching the appliance 10 to a user. In the embodiment shown, the connection member 20 is a hydrocolloid wafer for securing the appliance 10 to the skin of a user around their stoma. Alternatively, the connection member 20 may be for attaching the appliance 10 to a flange for attaching the appliance to a user (e.g. a two-piece appliance as it is known in the art).

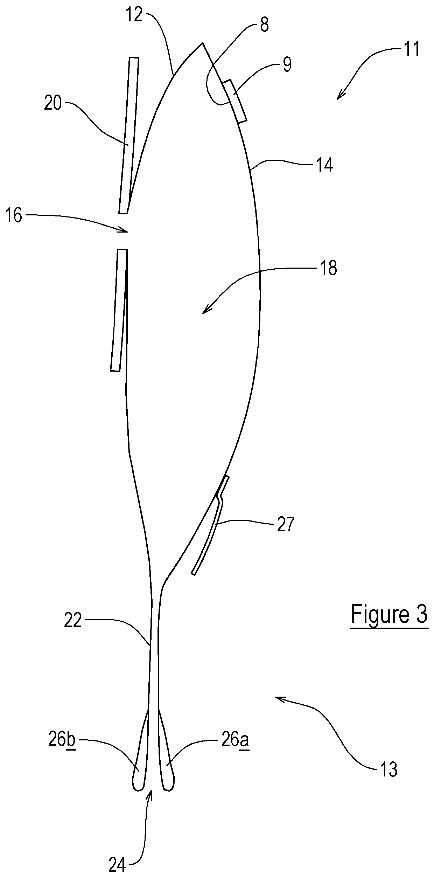

Referring to figure 3, the first and second walls 12, 14 define a waste collecting cavity 18 therebetween. In some embodiments the first and/or second walls 12, 14 may be covered by comfort layers though these are not essential. The appliance 10 may also have a filter 9 positioned over a suitable gas vent 8 in either or both of the first 12 or second 14 walls, as is well known in the art.

The lower end 13 of the appliance 10 is provided with an outlet 22 formed by portions of the first and second walls 12, 14, again, as is well known in the art. The outlet 22 extends downwardly away from the stoma-receiving opening 16 and terminates at an opening 24. The opening 24 permits waste collected in the collecting cavity 18 to be expelled from the appliance 10.

In the present embodiment first and second elongate stiffening members 26a, 26b are provided to assist with opening and closing of the opening 24. The stiffening members 26a, 26b are connected, e.g. adhered or welded, to the exterior surfaces of the first and second walls 12, 14, respectively, near the opening 24, such that they are positioned one on top of the other (when viewed from the front), e.g. they are positioned generally opposite each other. Both stiffening members extend laterally across the outlet 22 such that they extend generally perpendicularly to the direction in which waste exits the appliance 10, as shown in figures 1 and 3.

The stiffening members 26a, 26b need only be stiff in the sense that they are relatively stiff compared to the walls 12, 14 of the ostomy appliance 10. In this embodiment the stiffening members 26a, 26b are injection moulded from a plastics material.

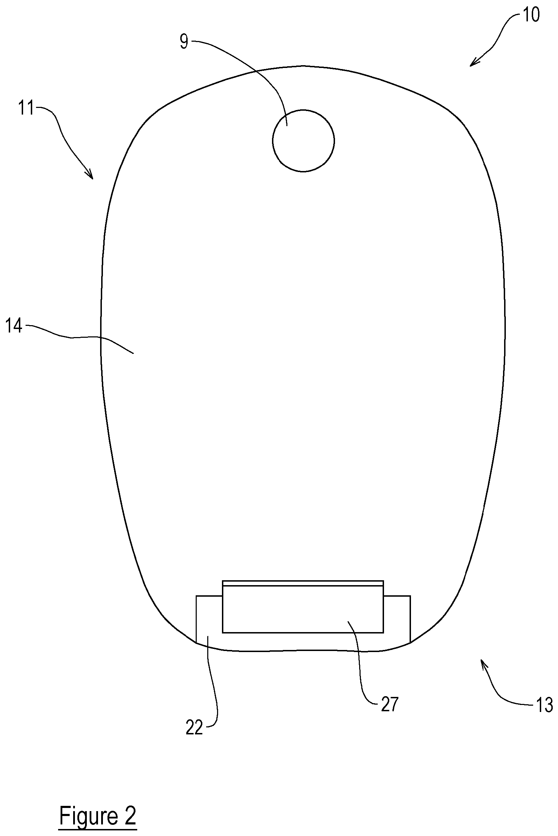

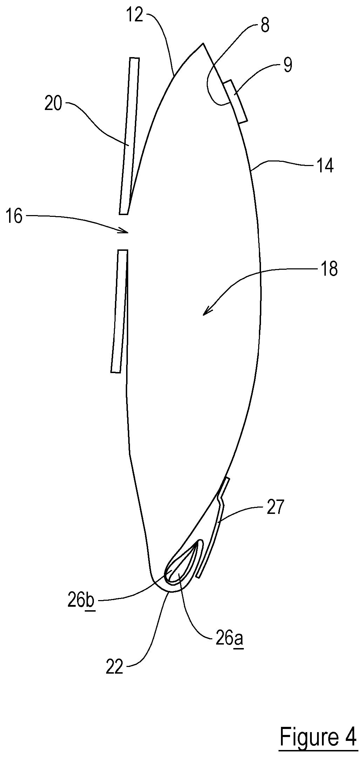

The outlet 22 may be stored, or stowed, by being rolled up around the stiffening members 26a, 26b, as is generally known in the art, and as shown in figures 2 and 4. Such rolling up uses the laterally extending edges of the members 26a, 26b as a fulcrum(s) to assist in tight closing of the outlet 24, to prevent leaking. In the present embodiment, the stiffening members 26a, 26b are rolled up two or three times, to seal the opening 24 in a closed condition. A flap 27 may be provided to secure the rolled up outlet 22 in its stowed condition. Any suitable fastening means, e.g. hook, and loop (Velcro) or adhesive, may be provided on respective faces of the flap 27 and outlet 22 to secure the outlet 22.

The stiffening members 26a, 26b are substantially identical to each other and are configured such that when one member 26a is rotated 180 degrees and positioned on top of the other member 26b, they mate and cooperate with one another. Thus, the facing surfaces of the members 26a, 26b cooperate with each other so as to substantially close the outlet 22 therebetween. As they are identical, only the shape of the stiffening member 26a will be discussed herein.

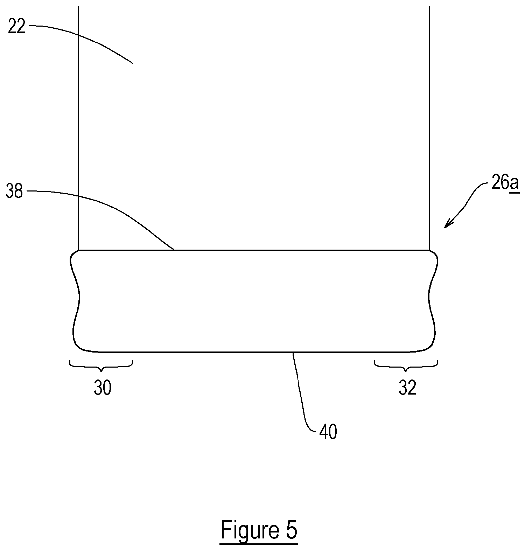

When looking at the member 26a from figure 5 the member 26a has first and second lateral end portions 30, 32 positioned at either side of the opening 24. First and second laterally extending edges or sides 38, 40 extend across the outlet 22 between the first and second end portions 30, 32, respectively. The first side 38 is positioned closer to the stoma-receiving opening than the second side 40.

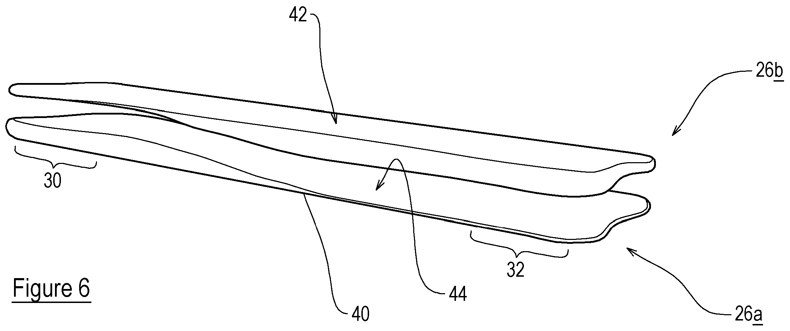

The stiffening member 26a is elongate and generally planar, e.g. flat, on its outer surface 42 which faces away from the outlet 22, as shown in figures 6, 7 and 8. However, a surface 44 of the member 26a which faces inwardly towards the outlet 22 (and towards the other member 26b) is contoured, or non-planar. This important shaping is described in more detail below.

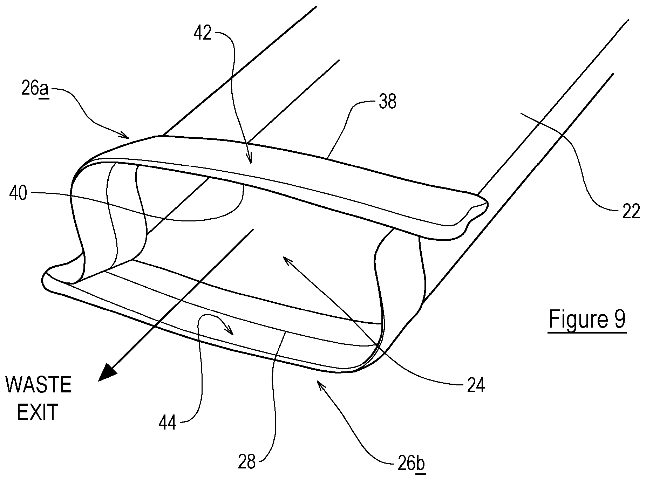

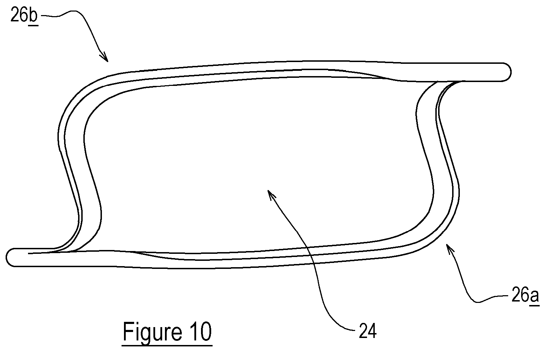

As shown in figures 6 to 10, a thickness of the stiffening member varies both between the first and second ends, or end portions, 30, 32 and between the first and second sides 38, 40. It should be noted that figures 6 to 8 show the stiffening members 26a, 26b not connected to their respective walls 12, 14 and with the stiffening members 26a, 26b spaced apart from each other along their entire length. This has been done to aid clarity and to show the contours of the inner, facing, surfaces of the members 26a, 26b. It should be noted that once the stiffening members 26a, 26b are connected to the walls 12, 14, the lateral ends thereof are essentially connected to each other, with the walls 12, 14 sandwiched therebetween, as show in figures 9 and 10.

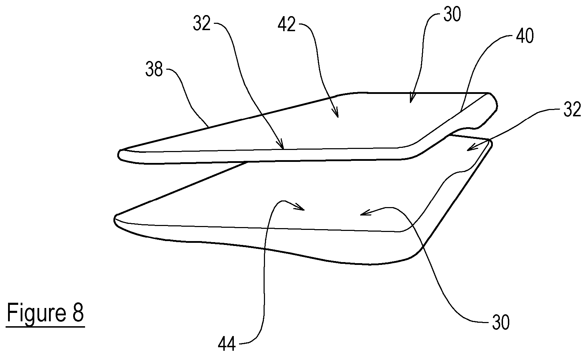

The thickness of the stiffening member 26a at its first end portion 30 positioned closest to the opening is greater than the thickness of the stiffening member 26a at its second end portion 32 positioned closest to the opening. By this we mean that a thickness of the stiffening member 26a decreases or reduces as it extends from the first end portion 20 to the second end portion 32. In the present embodiment, the reduction is a tapering of the thickness laterally from one side of the stiffening member 26a to the other, but it should also be noted that the degree of taper changes across the stiffening member 26a. Figure 8 highlights this point, in that the thickness of the end portion 30 increases as it extends from the edge 38 to the edge 40, but the thickness of the end portion 32 is substantially constant from the edge 38 to the edge 40.

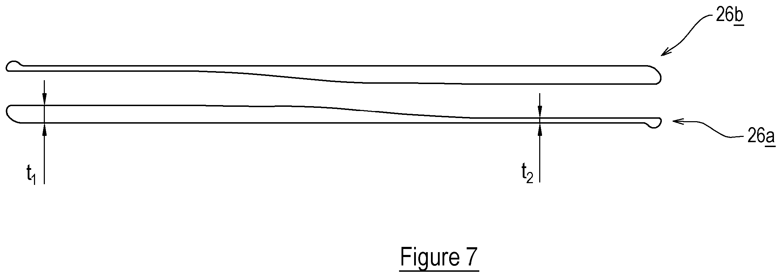

It follows therefore that the thickness of the edge 38 of each stiffening member 26a, 26b is substantially constant across the outlet. However, the thickness of the edge 40 of each stiffening member changes as that edge extends from one side of the stiffening member 26a, 26b to the other side of the stiffening member 26a, 26b (as is shown in figure 7). This change is represented on the figures by t₁ which is the thickness near the end 30 and t₂ which is the thickness near the end 32. The thickness of the edge 38 is also t₂.

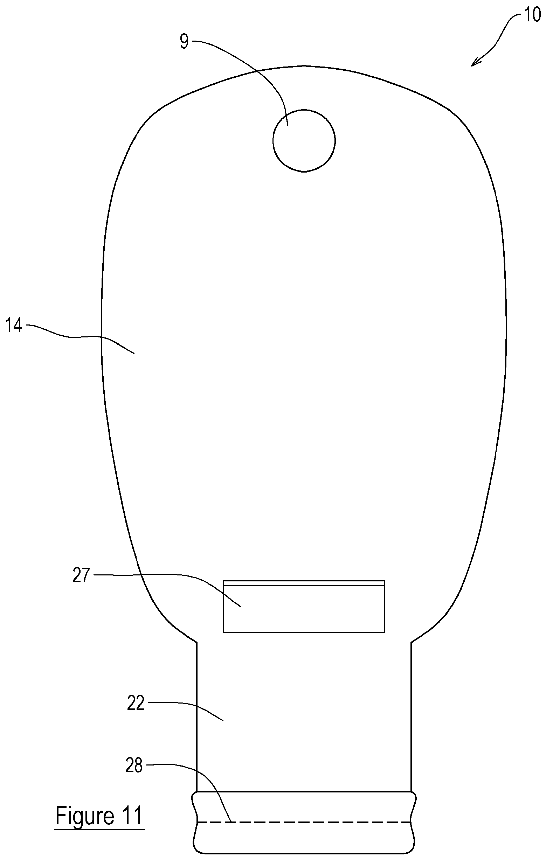

Referring to figures 11 to 12, the outlet 22 is formed from extensions or continuations of the first and second walls 12, 14 which terminate at a wall edge 28. In alternative embodiments the outlet 22 may be formed from only one continuous wall. Indeed, the outlet 22 may be formed separately from the remainder of the ostomy appliance 10 and welded or adhered to the remainder of the appliance 10 when it is manufactured.

Only a portion of each stiffening member 26a, 26b is connected to its respective first or second wall 12, 14. In other words, the stiffening members 26a, 26b are positioned such that they overlap the edges 28 of the first and second walls 12, 14 effectively extending the length of the outlet 22. In other words, the stiffening members 26a, 26b themselves at least partially define the opening 24.

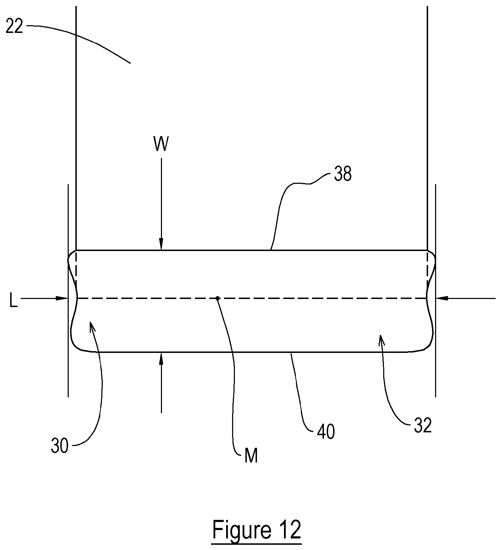

As shown in figure 12, the stiffening member 26a has a width W which extends between its first and second sides 38, 40. The stiffening member 26a also has a length L which extends between its first and second ends 30, 32. The stiffening member 26a is positioned such that up to 50% of the width W of the stiffening member 26a at the mid-point M (the mid-point being positioned halfway between the first and second ends 30, 32) extends over the edge 28. Other embodiments have been envisaged where either stiffening member 26a, 26b is positioned such that up to 10%, or up to 20%, or up to 30%, or up to 40%, or up to 50% of the width W of the stiffening member 26a, 26b at the mid-point M extends over the edge 28.

The wall edge 28 is substantially linear and extends generally perpendicularly across the outlet 22. The wall edge 28 may in some embodiments be curved or have a curved portion, e.g. it could be convex or concave.

To open the opening 24 of the appliance 10 the stiffening members 26a, 26b are bent away from each other by applying finger pressure to their first and second respective end portions 30, 32, such that they bow away from each other, as shown in figures 9 and 10. Once finger pressure is removed, the opening 24 moves to or substantially moves to its closed condition due to the stiffening members 26 being biased to a relatively flat position.

When the opening 24 is in a closed condition the inwardly facing surfaces 44 of the first and second stiffening members 26a, 26b cooperate with each other. In doing so, the outwardly facing surfaces 42 of the first and second stiffening members 26a, 26b are either parallel with each other or inclined at an angle to each other. In the present embodiment they are inclined to each other, i.e. the distance between the exterior surfaces increases as the surfaces extend from the edge 38 to the edge 40.

The stiffening members 26a, 26b can be bowed away from each other more easily compared to the prior art. This is because the stiffening members 26a, 26b bow more easily at their relatively thinner portions. This is beneficial because stiffening members can become stuck together, in use, and so by being designed to bow away from each other more easily the stiffening members 26a, 26b are able to more easily bow apart.

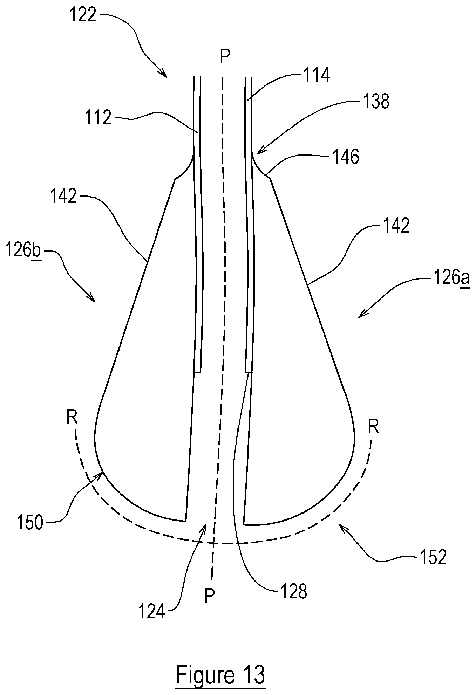

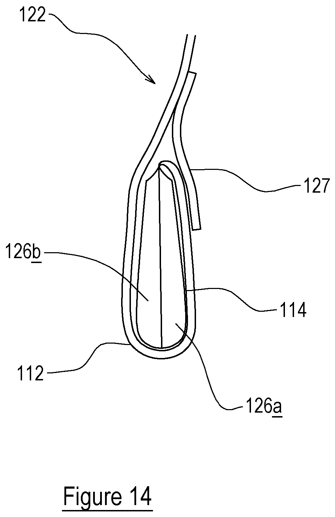

Figures 13 and 14 show the outlet of a second embodiment of an ostomy appliance in accordance with the present invention. Features which are in common with the first embodiment have been given the same reference numeral with the addition of 100. Those features will not be discussed again here.

The outwardly facing surfaces 142 of the first and second stiffening members 126a, 126b are generally planar, much like in the first embodiment. In this embodiment, as shown in figure 13, the inwardly facing surface 144 of each stiffening member 126a, 126b is substantially planar. In addition, the cross-sectional shape, e.g. profile, of each stiffening member 126a, 126b is substantially constant as it extends across the outlet 122. Together the first and second stiffening members 126a, 126b substantially form a teardrop shape, and are mirror images of each other about plane P. A first portion 146 of the outwardly facing surface 142 (positioned closest to the edge 138) is concave. This portion 146 connects to the generally planar surface 142 which in turn continues into an edge portion 152 which is positioned close to the opening 124 of the outlet 122. An exterior surface 150 of the edge portion is convex in the sense that it curves inwardly towards the opening 124.

The edge portions 152 abut or are positioned next to each other, when the opening 124 is closed and form a continuous curved profile. This is shown by the curve R on figure 13.

The exterior surface 150 of the edge portion 152 substantially prescribes a portion of a cylindrical surface, defined by the curve R. Thus, when the stiffening members 126a, 126b abut, their combined surfaces 150 substantially prescribe a larger portion of a cylindrical surface, e.g. about half of the surface of a cylinder.

The edge portions 152 are advantageous when rolling up the outlet 122 as it enables any waste trapped in the outlet 122 to be pushed out of the outlet against the edge portions 152. This ensures that a good seal is made and that a minimal quantity of waste can escape through the opening 124 when the outlet is rolled up as shown in figure 14.

Other alternative embodiments have been envisaged where only the stiffening member 126a or 126b is provided. In those embodiments the edge portion 152 of the stiffening member may separately form a continuous curved profile which follows a curve similar to R.

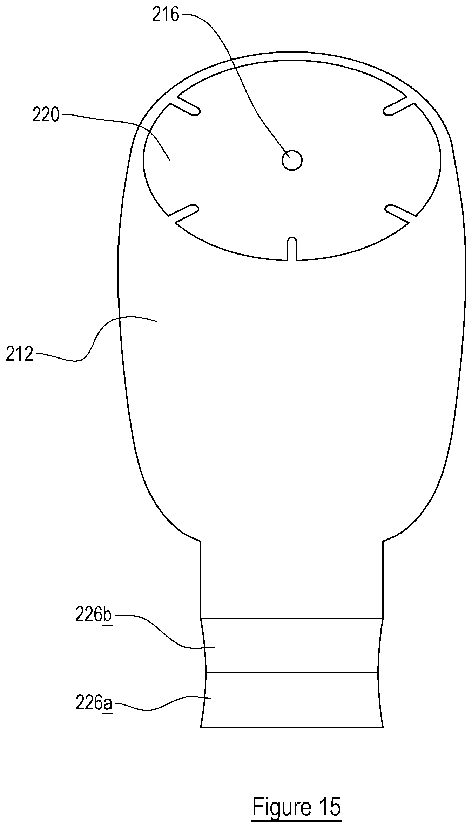

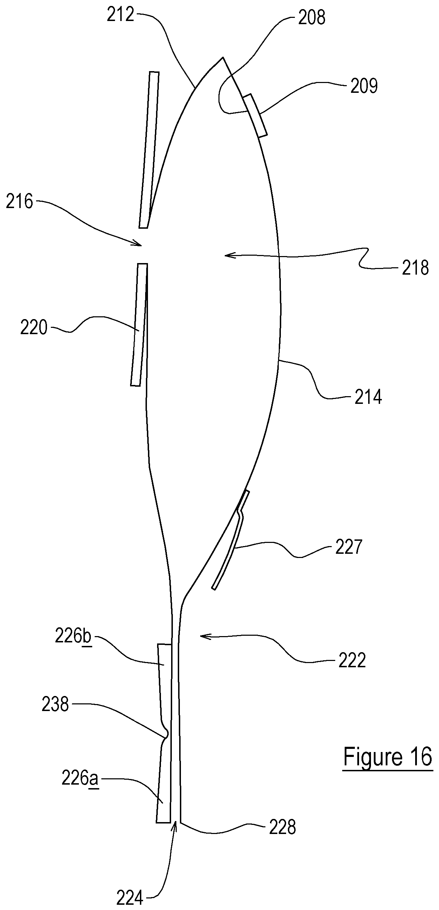

Figures 15 and 16 show a third embodiment of an ostomy appliance in accordance with the present invention. Features which are in common with the first embodiment have been given the same reference numeral with the addition of 200. Those features will not be discussed again here.

In the third embodiment the first and second stiffening members 226a, 226b are both provided on the first wall 212 (the wall which faces the user) of the outlet 222. The second stiffening member 226b is positioned above the first stiffening member 226a further away from the opening 224 of the outlet 222 than the first stiffening member 226a. In particular, the second stiffening member 226b abuts, or lies close to, the first side 238 of the first stiffening member 226a. The stiffening member 226a tapers, or narrows, as it extends upwardly away from the opening 222 whilst the opposite is true for the stiffening member 226b. The stiffening member 226b tapers, or narrows, in cross section as it extends downwardly towards the stiffening member 226a.

Preferably, no stiffening members are provided on the second wall 214 of the outlet 222. Therefore, the wall edge 228, or at least a portion of the wall edge 228, partially defines the opening 224 together with the lowermost edge of the stiffening member 226a. In this embodiment the surfaces 254 of the edge portions 252 do not follow a continuous curved profile, but they could be of a similar shape to the profile of the stiffening members in the second embodiment.

When it is desired to close the outlet, the first stiffening member 226a is rolled up until folds on to the wall 214, such that the stiffening members 226a, 226b are then positioned opposite each other. Both stiffening members 226a, 226b are then rolled up together, either one or more times and held in place by a flap similar to the flap 27 of the first embodiment.

In other alternative embodiments only a single stiffening member, for example only the first stiffening member 226a or the second stiffening member 226b, may be provided.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A drainable ostomy appliance (10, 110, 210) including:
first and second walls (12, 112, 212, 14, 114, 214) connected to each other at or near their peripheries, the first wall (12, 112, 212) having a stoma-receiving opening (16, 116, 216);
a collecting cavity (18, 118, 218) defined between the first and second walls;
a connection member (20, 120, 220) connected to the first wall for attaching the appliance to a user or for attaching the appliance to a flange for attaching the appliance to a user;
an outlet (22, 122, 222) which extends away from the stoma-receiving opening, the outlet having an opening (24, 124, 224) for waste and the outlet being formed from at least one wall which terminates at a wall edge (28, 128, 228); and
a first stiffening member (26a, 126a, 226a) which is connected to the wall forming the outlet, **characterised by** the first stiffening member being positioned such that it overlaps the edge of said wall effectively extending the length of the outlet.

2. A drainable ostomy appliance according to claim 1 wherein at least a portion of the wall edge (28, 128, 228) partially defines the opening (24, 124, 224).

3. A drainable ostomy appliance according to any preceding claim wherein the first stiffening member (26a, 126a, 226a) has first and second sides (38, 138, 238, 40, 140, 240), which extend across the outlet (22, 122, 222), and first and second ends (30, 130, 230, 32, 132, 232) which extend between the first and second sides; wherein the first side is closer to the stoma receiving opening (16, 116, 216) than the second side.

4. A drainable ostomy appliance according to claim 3 wherein the first stiffening member (26a, 126a, 226a) is positioned such that 10% of a width of the first stiffening member extending between the first and second sides (38, 138, 238, 40, 140, 240), at a mid-point M between the first and second ends (30, 130, 230, 32, 132, 232), extends over the edge (28, 128, 228) of the at least one wall.

5. A drainable ostomy appliance according to claim 3 wherein the first stiffening member (26a, 126a, 226a) is positioned such that 20% of a width of the first stiffening member extending between the first and second sides (38, 138, 238, 40, 140, 240), at a mid-point M between the first and second ends (30, 130, 230, 32, 132, 232), extends over the edge (28, 128, 228) of the at least one wall.

6. A drainable ostomy appliance according to claim 3 wherein the first stiffening member (26a, 126a, 226a) is positioned such that 30% of a width of the first stiffening member extending between the first and second sides (38, 138, 238, 40, 140, 240), at a mid-point M between the first and second ends (30, 130, 230, 32, 132, 232), extends over the edge (28, 128, 228) of the at least one wall.

7. A drainable ostomy appliance according to claim 3 wherein the first stiffening member (26a, 126a, 226a) is positioned such that 40% of a width of the first stiffening member extending between the first and second sides (38, 138, 238, 40, 140, 240), at a mid-point M between the first and second ends (30, 130, 230, 32, 132, 232), extends over the edge (28, 128, 228) of the at least one wall.

8. A drainable ostomy appliance according to claim 3 wherein the first stiffening member (26a, 126a, 226a) is positioned such that 50% of a width of the first stiffening member extending between the first and second sides (38, 138, 238, 40, 140, 240), at a mid-point M between the first and second ends (30, 130, 230, 32, 132, 232), extends over the edge (28, 128, 228) of the at least one wall.

9. A drainable ostomy appliance according to any one of claims 4 to 8 wherein the mid-point M is half way between the first and second ends (30, 130, 230, 32, 132, 232).

10. A drainable ostomy appliance according to any preceding claim wherein the outlet (24, 124, 224) is formed by an extension or continuation of the first and second walls (12, 112, 212, 14, 114, 214) which define the waste collecting cavity (18, 118, 218).

11. A drainable ostomy appliance according to any preceding claim including a second stiffening member (26b, 126b, 226b), including one or more or all of the features of the first stiffening member (26a, 126a, 226a) as set forth in claims 1 to 10.

12. A drainable ostomy appliance according to claim 11 when dependent on claim 10 wherein the first and second stiffening members (226a, 226b) are positioned on the first wall (212) and wherein the second stiffening member (226b) is positioned above the first stiffening member (226a) further from the opening (224) of the outlet (222) than the first stiffening member (226a).

13. A drainable ostomy appliance according to claim 12 wherein the second stiffening member (226b) abuts or lies close to the first side (238) of the first stiffening member (226a).

14. A drainable ostomy appliance according to claim 11 wherein the second stiffening member (226b) is positioned generally opposite the first stiffening member (226a).

15. A drainable ostomy appliance according to any one of claims 11 to 14 wherein the second stiffening member (226b) is substantially the same as the first stiffening member (226a).

## Patentansprüche

1. Entleerbares Ostomiegerät (10, 110, 210), umfassend:
Erste und zweite Wände (12, 112, 212, 14, 114, 214), die miteinander an oder nahe ihrer Peripherien verbunden sind, wobei die erste Wand eine Stoma-Aufnahmeöffnung (16) aufweist; und
einen Sammelhohlraum (18, 118, 218), der zwischen den ersten und zweiten Wänden definiert ist;
ein mit der ersten Wand verbundenes Verbindungselement (20, 120, 220) zum Befestigen des Geräts an einem Benutzer oder zum Befestigen des Geräts an einem Flansch zum Befestigen des Geräts an einem Benutzer;
einen Auslass (22, 122, 222), der sich von der Stoma-Aufnahmeöffnung weg erstreckt, wobei der Auslass eine Öffnung (24, 124, 224) für Abfall aufweist und aus mindestens einer Wand gebildet ist, die an einer Wandkante (28, 128, 228) endet; und
ein Versteifungselement (26a, 126a, 226a), das mit der Wand verbunden ist, die den Auslass bildet, **dadurch gekennzeichnet, dass** das erste Versteifungselement so positioniert ist, dass es die Kante der Wand überlappt, welches die Länge des Auslasses effektiv verlängert.

2. Entleerbares Ostomiegerät nach Anspruch 1, wobei mindestens ein Abschnitt der Wandkante (28, 128, 228) teilweise die Öffnung (24, 124, 224) definiert.

3. Entleerbares Ostomiegerät nach einem der vorhergehenden Ansprüche, wobei das erste Versteifungselement (26a, 126a, 226a) erste und zweite Seiten (38, 138, 238, 40, 140, 240) aufweist, die sich über den Auslass (22, 122, 222) erstrecken, sowie erste und zweite Enden (30, 130, 230, 32, 132, 232) aufweist, die sich zwischen den ersten und zweiten Seiten erstrecken; wobei die erste Seite näher an der Stoma-Aufnahmeöffnung (16, 116, 216) als die zweite Seite liegt.

4. Entleerbares Ostomiegerät nach Anspruch 3, wobei das Versteifungselement (26a, 126a, 226a) so positioniert ist, dass 10 % einer Breite des ersten Versteifungselements, das sich zwischen den ersten und zweiten Seiten (38, 138, 238, 40, 140, 240) erstreckt, an einem Mittelpunkt M zwischen den ersten und zweiten Enden (30, 130, 230, 32, 132, 232) über die Kante (28, 128, 228) der mindestens einen Wand hinausragt.

5. Entleerbares Ostomiegerät nach Anspruch 3, wobei das erste Versteifungselement (26a, 126a, 226a) so positioniert ist, dass 20 % einer Breite des ersten Versteifungselements, das sich zwischen den ersten und zweiten Seiten (38, 138, 238, 40, 140, 240) erstreckt, an einem Mittelpunkt M zwischen dem ersten und dem zweiten Ende (30, 130, 230, 32, 132, 232) über die Kante (28, 128, 228) der mindestens einen Wand hinausragen.

6. Entleerbares Ostomiegerät nach Anspruch 3, wobei das Versteifungselement (26a, 126a, 226a) so positioniert ist, dass 30 % einer Breite des ersten Versteifungselements, das sich zwischen den ersten und zweiten Seiten (38, 138, 238, 40, 140, 240) erstreckt, an einem Mittelpunkt M zwischen den ersten und zweiten Enden (30, 130, 230, 32, 132, 232) über die Kante (28, 128, 228) der mindestens einen Wand hinausragt.

7. Entleerbares Ostomiegerät nach Anspruch 3, wobei das erste Versteifungselement (26a, 126a, 226a) so positioniert ist, dass 40 % einer Breite des ersten Versteifungselements, das sich zwischen den ersten und zweiten Seiten (38, 138, 238, 40, 140, 240) erstreckt, an einem Mittelpunkt M zwischen den ersten und zweiten Enden (30.130, 130, 230, 32, 132, 232) über die Kante (28, 128, 228) der mindestens einen Wand hinausragt.

8. Entleerbares Ostomiegerät nach Anspruch 3, wobei das erste Versteifungselement (26a, 126a, 226a) so positioniert ist, dass 50 % einer Breite des ersten Versteifungselements, das sich zwischen den ersten und zweiten Seiten (38, 138, 238, 40, 140, 240) erstreckt, an einem Mittelpunkt M zwischen den ersten und zweiten Enden (30, 130, 230, 32, 132, 232) über die Kante (28, 128, 228) der mindestens einen Wand hinausragt.

9. Entleerbares Ostomiegerät nach einem der Ansprüche 4 bis 8, wobei der Mittelpunkt M auf halbem Weg zwischen den ersten und zweiten Enden (30,130, 230, 32, 132, 232) liegt.

10. Entleerbares Ostomiegerät nach einem vorhergehenden Anspruch, wobei der Auslass (24, 124, 224) durch eine Verlängerung oder Fortsetzung der ersten und zweiten Wände (12, 112, 212, 14, 114, 214) gebildet wird, die den Abfallsammelhohlraum (18, 118, 218) definieren.

11. Entleerbares Ostomiegerät nach einem vorhergehenden Anspruch, das ein zweites Versteifungselement (26b, 126b, 226b) umfasst, das ein oder mehrere oder alle Merkmale des ersten Versteifungselements (26a, 126a, 226a) wie in den Ansprüchen 1 bis 10 dargelegt umfasst.

12. Entleerbares Ostomiegerät nach Anspruch 11 in Abhängigkeit von Anspruch 10, wobei die ersten und zweiten Versteifungselemente (226a, 226b) an der ersten Wand (212) positioniert sind und wobei das zweite Versteifungselement (226b) oberhalb des ersten Versteifungselements (226a) und weiter von der Öffnung (224) des Auslasses (222) entfernt positioniert ist als das erste Versteifungselement (226a).

13. Entleerbares Ostomiegerät nach Anspruch 12, wobei das zweite Versteifungselement (226b) an der ersten Seite (238) des ersten Versteifungselements (226a) anliegt oder nahe dieser liegt.

14. Entleerbares Ostomiegerät nach Anspruch 11, wobei das zweite Versteifungselement (226b) generell gegenüber dem ersten Versteifungselement (226a) positioniert ist.

15. Entleerbares Ostomiegerät nach einem der Ansprüche 11 bis 14, wobei das zweite Versteifungselement (226b) im Wesentlichen dasselbe wie das erste Versteifungselement (226a) ist.

## Revendications

1. Appareil de stomie drainable (10, 110, 210) comprenant :
des première et deuxième parois (12, 112, 212, 14, 114, 214) reliées l'une à l'autre au niveau ou à proximité de leur périphérie, la première paroi (12, 112, 212) comportant une ouverture de réception de stomie (16, 116, 216) ;
une cavité de collecte (18, 118, 218) délimitée entre les première et deuxième parois ;
un élément de liaison (20, 120, 220) relié à la première paroi pour fixer l'appareil à un utilisateur ou pour fixer l'appareil à une bride pour fixer l'appareil à un utilisateur ;
une sortie (22, 122, 222) qui s'étend à partir de l'ouverture de réception de stomie, la sortie comportant une ouverture (24, 124, 224) pour les déchets et la sortie étant formée d'au moins une paroi qui se termine par un bord de paroi (28, 128, 228) ; et
un premier élément de raidissement (26A, 126A, 226A) qui est relié à la paroi formant la sortie, **caractérisé en ce que** le premier élément de raidissement est positionné de telle sorte qu'il chevauche le bord de ladite paroi en augmentant effectivement la longueur de la sortie.

2. Appareil de stomie drainable selon la revendication 1, dans lequel au moins une partie du bord de paroi (28, 128, 228) délimite partiellement l'ouverture (24, 124, 224).

3. Appareil de stomie drainable selon l'une quelconque des revendications précédentes, dans lequel le premier élément de raidissement (26a, 126a, 226a) comporte des premier et deuxième côtés (38, 138, 238, 40, 140, 240), qui s'étendent à travers la sortie (22, 122, 222), et des première et deuxième extrémités (30, 130, 230, 32, 132, 232) qui s'étendent entre les premier et deuxième côtés ; dans lequel le premier côté est plus proche de l'ouverture de réception de stomie (16, 116, 216) que le deuxième côté.

4. Appareil de stomie drainable selon la revendication 3, dans lequel le premier élément de raidissement (26a, 126a, 226a) est positionné de telle sorte que 10 % d'une largeur du premier élément de raidissement s'étendant entre les premier et deuxième côtés (38, 138, 238, 40, 140, 240), à un point médian M entre les première et deuxième extrémités (30, 130, 230, 32, 132, 232), s'étende par-dessus le bord (28, 128, 228) de l'au moins une paroi.

5. Appareil de stomie drainable selon la revendication 3, dans lequel le premier élément de raidissement (26a, 126a, 226a) est positionné de telle sorte que 20 % d'une largeur du premier élément de raidissement s'étendant entre les premier et deuxième côtés (38, 138, 238, 40, 140, 240), à un point médian M entre les première et deuxième extrémités (30, 130, 230, 32, 132, 232), s'étende par-dessus le bord (28, 128, 228) de l'au moins une paroi.

6. Appareil de stomie drainable selon la revendication 3, dans lequel le premier élément de raidissement (26a, 126a, 226a) est positionné de telle sorte que 30 % d'une largeur du premier élément de raidissement s'étendant entre les premier et deuxième côtés (38, 138, 238, 40, 140, 240), à un point médian M entre les première et deuxième extrémités (30, 130, 230, 32, 132, 232), s'étende par-dessus le bord (28, 128, 228) de l'au moins une paroi.

7. Appareil de stomie drainable selon la revendication 3, dans lequel le premier élément de raidissement (26a, 126a, 226a) est positionné de telle sorte que 40 % d'une largeur du premier élément de raidissement s'étendant entre les premier et deuxième côtés (38, 138, 238, 40, 140, 240), à un point médian M entre les première et deuxième extrémités (30, 130, 230, 32, 132, 232), s'étende par-dessus le bord (28, 128, 228) de l'au moins une paroi.

8. Appareil de stomie drainable selon la revendication 3, dans lequel le premier élément de raidissement (26a, 126a, 226a) est positionné de telle sorte que 50 % d'une largeur du premier élément de raidissement s'étendant entre les premier et deuxième côtés (38, 138, 238, 40, 140, 240), à un point médian M entre les première et deuxième extrémités (30, 130, 230, 32, 132, 232), s'étende par-dessus le bord (28, 128, 228) de l'au moins une paroi.

9. Appareil de stomie drainable selon l'une quelconque des revendications 4 à 8, dans lequel le point médian M est à mi-chemin entre les première et deuxième extrémités (30, 130, 230, 32, 132, 232).

10. Appareil de stomie drainable selon l'une quelconque des revendications précédentes, dans lequel la sortie (24, 124, 224) est formée d'un prolongement ou d'une continuation des première et deuxième parois (12, 112, 212, 14, 114, 214) qui délimitent la cavité de collecte des déchets (18, 118, 218).

11. Appareil de stomie drainable selon l'une quelconque des revendications précédentes, comprenant un deuxième élément de raidissement (26b, 126b, 226b), comprenant une ou plusieurs ou la totalité des caractéristiques du premier élément de raidissement (26a, 126a, 226a) telles qu'énoncées dans les revendications 1 à 10.

12. Appareil de stomie drainable selon la revendication 11 lorsque celle-ci est dépendante de la revendication 10, dans lequel les premier et deuxième éléments de raidissement (226a, 226b) sont positionnés sur la première paroi (212) et dans lequel le deuxième élément de raidissement (226b) est positionné au-dessus du premier élément de raidissement (226a) plus loin de l'ouverture (224) de la sortie (222) que le premier élément de raidissement (226a).

13. Appareil de stomie drainable selon la revendication 12, dans lequel le deuxième élément de raidissement (226b) se trouve contre le ou à proximité du premier côté (238) du premier élément de raidissement (226a).

14. Appareil de stomie drainable selon la revendication 11, dans lequel le deuxième élément de raidissement (226b) est positionné d'une manière générale à l'opposé du premier élément de raidissement (226a).

15. Appareil de stomie drainable selon l'une quelconque des revendications 11 à 14, dans lequel le deuxième élément de raidissement (226b) est sensiblement identique au premier élément de raidissement (226a).
